# EUROPÄISCHE PATENTSCHRIFT

(11) **EP 0 954 256 B1**
(45) Veröffentlichungstag und Bekanntmachung des Hinweises auf die Patenterteilung: **20.08.2003**
(21) Anmeldenummer: 97952044.2
(22) Anmeldetag: 09.12.1997
(51) Int. Cl.: A61F 2/20

(54) **SHUNT-VENTIL**
SHUNT VALVE
VALVE DE DERIVATION

(30) Priorität: 16.12.1996 DE 19651951
(43) Veröffentlichungstag der Anmeldung: 10.11.1999
(73) Patentinhaber: Adeva Medical GmbH & Co., 23556 Lübeck (DE)
(72) Erfinder: GRUNDEI, Hans, D-23558 Lübeck (DE)
(74) Vertreter: Fuchs Mehler Weiss & Fritzsche
(86) Internationale Anmeldenummer: EP9706852
(87) Internationale Veröffentlichungsnummer: WO98026734

(56) Entgegenhaltungen:
- EP-A- 0 222 509
- EP-A- 0 299 705
- EP-A- 0 651 980
- WO-A-97/45075

## Beschreibung

Die vorliegende Erfindung betrifft ein sogenanntes Shunt-Ventil, welches bekanntermaßen nach einer an einem Patienten durchgeführten Laryngektomie in einen operativ erzeugten Verbindungskanal zwischen Trachea und Oesophagus, den sogenannten Shunt, zum Einsatz kommt, um es dem Patienten zu ermöglichen, neben der lebensnotwendigen Luftführung durch ein operativ erzeugtes Tracheostoma auch eine Luftführung zur Tonbildung, also zum Sprechen vorzunehmen. Verschließt der Patient das Tracheostoma beim Ausatmen mit der Hand oder wird das Tracheostoma bei Überschreiten eines vorbestimmbaren Druckes durch ein Tracheostoma-Ventil verschlossen, kann durch den künstlich erzeugten Verbindungskanal Luft aus der Trachea in den Oesophagus treten und zur Lautbildung verwendet werden.

Shunt-Ventile dienen in erster Linie dazu, den künstlichen Verbindungskanal offen zu halten. Darüber hinaus aber haben sie eine weitere wichtige Funktion, nämlich Sekrete und Speisereste vom Oesophagus nicht in die Trachea treten zu lassen, was nämlich anderenfalls größte Komplikationen nach sich ziehen würde. Hierzu sind einseitig wirkende Ventile auf die Oesophagusseite des Shunt-Ventiles vorgeschlagen worden, beispielsweise in der DD-A-275 183 in Form eines Klappenventils oder gemäß der DE-A-32 11 126 in der Ausbildung eines oesophagusseitig an sich geschlossenen Rohres, welches nur über einen Ventilschlitz verfügt, durch welchen erwünschtermaßen Luft von der Trachea in den Oesophagus geleitet werden kann.

Die bislang vorgeschlagenen Lösungen haben aber nur einen suboptimalen Abdichtungseffekt und überzeugen in der Praxis kaum. So kommt es immer wieder vor, daß bei den bekannten Shunt-Ventilen Speisereste oder Sekrete in die Trachea gelangen, was dem Patienten größte Schwierigkeiten bereiten kann.

Abhilfe schafft hier auch nicht eine Stimmprothese gattungsgemäßer Ausbildung gemäß der FR 2494581 A, die eine Stimmprothese zum Einsatz in einen operativ erzeugten Verbindungskanal zwischen Trachea und Oesophagus zeigt, die ein röhrenförmiges Teil mit einem der Trachea zuzuwendenden freien Lumen und einem dem Oesophagus zuzuwendenden, durch ein einseitig wirkendes Klappenventil verschließbaren Lumen, dessen Klappe bei fehlenden Gegenkräften in seiner Verschlußstellung verharrt, und welche beim Ausatmen unter Öffnung der Klappe einen Luftstrom von der Trachea in den Oesophagus zuläßt. Am oesophagusseitigen Ende ist es nicht auszuschließen, daß Speisereste in das röhrenförmige Teil eintreten und so das Lumen des Shunt-Ventils verstopfen oder sogar in die Trachea übertreten können, wo sie schwere Hustenanfälle bis hin zu Erstickungsfällen verursachen können.

Aus der US-A-4,820,304 ist eine weitere Stimmprothese bekannt, bei der vorgeschlagen wird, am Shunt-Ventil oesophagusseitig eine dachförmige Überdeckung anzuformen, die einem Eintritt von Speiseresten in das Ventilinnere entgegenwirkt.

Vor dem aufgezeigten Hintergrund ist es nun die Aufgabe der vorliegenden Erfindung, hier Abhilfe zu schaffen, d.h. ein Shunt-Ventil anzugeben, welches hinsichtlich der Abdichtungsfunktion der Trachea vom Oesophagus eine entscheidende Verbesserung gegenüber den bekannten Lösungen aufweist.

Gelöst wird diese Aufgabe durch ein Shunt-Ventil gemäß dem Anspruch 1. Weitere vorteilhafte Ausgestaltungen sind in den Unteransprüchen angegeben. Demnach wird in Weiterbildung des gattungsgemäßen Shunt-Ventils vorgeschlagen,

daß der Schwenkbereich der Klappe des Klappenventils vollständig von einer dachförmigen Ausstülpung der Kunststoffhülle überdeckt ist, und

daß eine auf der Klappe lastende Federkraft von einer im dachförmigen Überdeckungsbereich der Kunststoffhülle angeformten, die Klappe direkt mit einem Ende berührenden Rückstellfeder erzeugt wird, welche die Klappe in die Verschlußstellung drängt.

Die Realisierung des röhrenförmigen Teils führt dazu, daß das eigentliche Kernstück des Shunt-Ventils, nämlich das röhrenförmige Teil, quasi eine Armierung für die Kunststoffhülle, die beispielsweise aus Silikon bestehen kann, bildet. Die Körperverträglichkeit wird so deutlich verbessert. Zudem läßt sich der innere Röhrenteil zu Reinigungszwecken aus der Kunststoffhülle herausziehen, wobei die Kunststoffhülle in situ verbleiben kann.

Die dachförmige Ausstülpung der Kungststoffhülle und die Rückstellfeder führen zu einer deutlichen Erhöhung der Sicherheit des erfindungsgemäßen Shunt-Ventils. Die Lösung der angeformten Rückstellfeder ist im übrigen besonders kostengünstig herzustellen, da die Rückstellfeder einen einstückigen Bestandteil der übrigen Kunststoffhülle bilden kann.

Das aktive Verschließen des Klappenventils durch die auf die Klappe des Ventils wirkenden Rückstellkräfte der Rückstellfeder bietet eine wesentlich höhere Sicherheit als die passiv wirkenden Ventilglieder der bekannten Shunt-Ventile, so daß mit den herkömmlichen Komplikationen beim Tragen des erfindungsgemäßen Shunt-Ventils nicht zu rechnen ist.

Die Einbaulage des Shunt-Ventil sollte so sein, daß die Klappe nach caudal hin öffnet, wohingegen die dachförmige Ausstülpung nach kranial hin quasi eine Abschirmung bildet.

Vorzugsweise besteht der innere röhrenförmige Teil aus einem körperverträglichen Metall. Dies bietet die Möglichkeit einer hinreichenden mechanischen Stabilität, wobei auch die Klappe des oesophagusseitigen Klappenventils aus Metall bestehen kann und eine Kegelstumpfform aufweist, um eine optimale Abdichtung zu erzielen.

Die Stabilität der Lage des Shunt-Ventiles in dem Verbindungskanal zwischen Trachea und Oesophagus kann vorteilhaft dadurch erhöht werden, daß an der Kunststoffhülle zwei umlaufende Flansche angeformt sind, zwischen denen die Oesophagus- und die Tracheawand zu liegen kommen. Letztere werden im Bereich des Verbindungskanals operativ miteinander verbunden, beispielsweise vernäht, so daß beide Wände zusammen zwischen den Flanschen liegen.

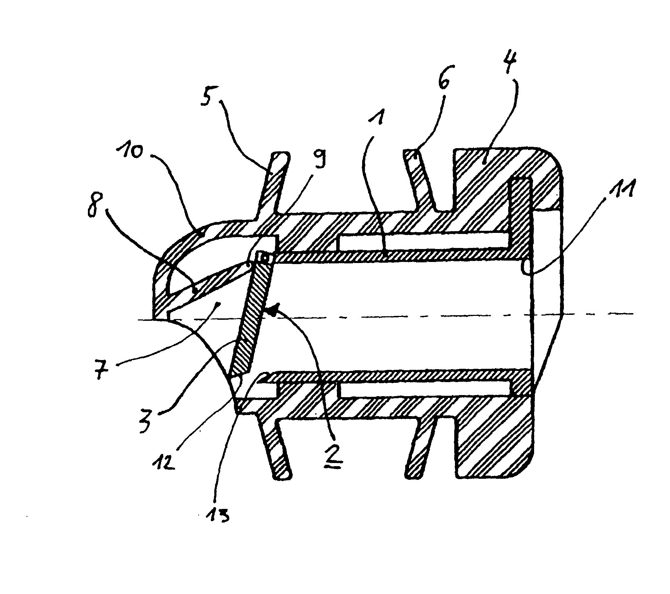

Die Erfindung wird nachfolgend anhand eines Ausführungsbeispiels gemäß der einzigen Zeichnungsfigur erläutert. Diese zeigt den Längsschnitt durch die besonders bevorzugte Ausführungsform.

Vorliegend ist das röhrenförmige Teil, welches in den operativ erzeugten Verbindungskanal zwischen Trachea und Oesophagus gesetzt wird, mit dem Bezugszeichen 1 bezeichnet. Tracheaseitig weist das röhrenförmige Teil 1 ein freies Lumen 11 auf, welches einen hinreichend großen Luftstrom von der Trachea hinein in den Oesophagus zuläßt. Oesophagusseitig ist ein Klappenkegelventil 2 angeordnet, welches mit seiner im wesentlichen kegelstumpfförmigen Klappe 3 das freie Lumen des röhrenförmigen Teils 1 vollständig verschließen kann, wenn keine Luftströmung vom freien Lumen 11 hin zur Ventilklappe aktiv ist, also wenn keine der die Klappe 3 in die Verschlußstellung drängenden Kraft entgegenwirkende Kraft wirkt. Das röhrenförmige Teil 1 wird vorzugsweise so in den Verbindungskanal zwischen Trachea und Oesophagus gesetzt, daß die Klappe 3 nach kaudal hin öffnet, um so einer eventuellen Gefahr des Eintretens von Speiseresten oder Sekreten in das Lumen des röhrenförmigen Teils 1 sicher zu verhindern.

Die kegelstumpfförmige Ausbildung der Klappe 3 ist durch die Fase 12 angedeutet, die ihr Gegenstück in der Fase 13 am röhrenförmigen Teil 1 findet, um eine optimale Abdichtung zu erreichen.

Das röhrenförmige Teil 1 ist mit einer Kunststoffhülle 4 versehen, welche vorliegend als eigenständiges Mantelteil dargestellt ist. Auf ihrer Peripherie weist die Kunststoffhülle 4 zwei umlaufende Flansche 5 und 6 auf, zwischen denen nach Einsetzen des Ventils in den Shunt die Oesophagus- und die Tracheawand zu liegen kommen.

Rückseitig, d.h. oesophagusseitig, setzt sich der Hüllenteil 4 in einer dachförmigen Ausstülpung 10 fort, die den Schwenkbereich der Klappe 3 des Klappenventils 2 vollständig überdeckt und quasi ein schützendes Dach über dem Ventil 2 ausbildet, insbesondere dann, wenn das Shunt-Ventil 1 so eingesetzt ist, daß die Ausstülpung 10 nach kranial hin abschirmt.

Die Ausstülpung 10 dient aber nicht nur der Abschirmung gegenüber Speiseresten, etc, sondern auch zur Halterung der Rückstellfeder 8 im hinteren Bereich. Vorliegend ist die Rückstellfeder 8 als ein einstückig mit dem Überzug 4 ausgebildetes Element, welches die Klappe 3 des Ventils 2 mit einer Rückstell- oder Rückschwenkkraft beaufschlagt, dargestellt. Es weist eine solche Länge auf, daß es mit seinem einen Ende 9 direkt an der Klappe 3 anliegt.

Wenn der Patient einen Luftstrom von der Trachea in den Oesophagus leitet, hebt die Klappe 3 entgegen der Federkraft der Rückstellfeder 8 von dem röhrenförmigen Teil 1 ab und schwenkt in den Schwenkbereich 7 hinein. Bei nachlassendem Luftstrom oder Ausbleiben des Luftstroms bewirkt die Rückstellfeder 8, daß die Klappe 3 unverzüglich das freie Lumen des röhrenförmigen Teils 1 wieder abdichtet.

Der Hüllenteil 4 besteht vorwiegend aus Silikon, der in seiner Shorehärte einstellbar ist, so, daß die Rückstellkraft von der Rückstellfeder 8 auf das Schwenken der Klappe 3 so erfolgt, daß es keinerlei Anstrengung bedarf, die Klappe 3 vom röhrenförmigen Teil 1 beim Ausatmen abzuschwenken.

## Patentansprüche

1. Shunt-Ventil zum Einsatz in einen operativ erzeugten Verbindungskanal zwischen Trachea und Oesophagus, aufweisend ein röhrenförmiges Teil (1) mit einem tracheaseitigen freien Lumen (11) welches mit einem oesophagusseitig am röhrenförmigen Teil (1) angeordneten Klappenventil (2) verschließbar ist, dessen Klappe (3) bei fehlenden Gegenkräften in seiner Verschlußstellung verharrt, und welches geeignet ist, unter Öffnung der Klappe (3) einen Luftstrom von der Trachea in den Oesophagus zuzulassen, wobei das röhrenförmige Teil mit einer körperverträglichen Kunststoffhülle versehen ist, **dadurch gekennzeichnet, daß**
- der Schwenkbereich (7) der Klappe (3) des Klappenventils (2) vollständig von einer dachförmigen Ausstülpung (10) der Kunststoffhülle (4) überdeckt ist, und daß
- eine auf der Klappe (3) lastende Federkraft von einer im dachförmigen Überdeckungsbereich der Kunststoffhülle (4) angeformten, die Klappe (3) direkt mit einem Ende (II) berührenden Rückstellfeder (8) erzeugt wird, welche die Klappe in die Verschlußstellung drängt.

2. Shunt-Ventil nach Anspruch 1, bei dem das röhrenförmige Teil (1) aus einem körperverträglichen Metall besteht.

3. Shunt-Ventil nach Anspruch 1 oder 2, bei dem an der Kunststoffhülle (4) zwei umlaufende Flansche (5, 6) angeformt sind, zwischen denen die Oespphagus- und Tracheawand zu liegen kommen.

4. Shunt-Ventil nach einem der Ansprüche 1 bis 3, bei dem die Kunststoffhülle (4) aus Silikon besteht.

## Claims

1. Shunt valve for use in a connecting channel produced by operation between trachea and oesophagus, having a tubular part (1) with a trachea-side free lumen (11), which can be closed by a flap valve (2) arranged on the tubular part (1) on the oesophagus-side, the flap (3) of the flap valve (2) remaining in its closed position in the absence of counter-forces, and which is suitable to admit an air stream from the trachea into the oesophagus while opening the flap (3), wherein the tubular part is provided with a biocompatible plastic sheath, **characterised in that**
- the pivoting region (7) of the flap (3) of the flap valve (2) is covered completely by a roof-shaped protuberance (10) of the plastic sheath (4), and **in that**
- a spring force bearing on the flap (3) is produced by a restoring spring (8) contacting the flap (3) directly with one end (11) and moulded on in the roof-shaped covering region of the plastic sheath (4), and which presses the flap into the closed position.

2. Shunt valve according to claim 1, in which the tubular part (1) consists of a biocompatible metal.

3. Shunt valve according to claim 1 or 2, in which two annular flanges (5, 6), between which the oesophagus and trachea wall come to rest, are moulded onto the plastic sheath (4).

4. Shunt valve according to one of claims 1 to 3, in which the plastic sheath (4) consists of silicone.

## Revendications

1. Vanne de dérivation pour une utilisation dans un canal de liaison entre la trachée et l'oesophage, lequel est réalisé à l'aide d'une opération, comprenant une partie tubulaire (1) avec une cavité creuse libre (11), qui est située du côté de la trachée et qui peut être obturée à l'aide d'une vanne à clapet (2) disposée du côté de l'oesophage sur la partie tubulaire (1), dont le clapet (3) reste dans sa position d'obturation en l'absence d'efforts opposés et laquelle est, avec l'ouverture du clapet (3), adaptée pour permettre un passage d'air depuis la trachée jusque dans l'oesophage, la partie tubulaire étant munie d'une enveloppe en matériau synthétique compatible avec l'organisme, **caractérisée**
- **en ce que** la zone de pivotement (7) du clapet (3) de la vanne à clapet (2) est entièrement recouverte par une protubérance en forme de toit (10) de l'enveloppe en matériau synthétique (4) et
- **en ce qu'**une force de ressort agissant sur le clapet (3) est produite par un ressort de rappel (8) formé sur la zone de recouvrement en forme de toit de l'enveloppe en matériau synthétique (4), qui touche directement le clapet (3) avec une extrémité (11) et qui repousse le clapet dans la position d'obturation.

2. Vanne de dérivation selon la revendication 1, **caractérisée en ce que** la partie tubulaire (1) est constituée d'un métal compatible avec l'organisme.

3. Vanne de dérivation selon la revendication 1 ou 2, **caractérisée en ce que** sur l'enveloppe en matériau synthétique (4) sont formées deux brides circulaires (5, 6) entre lesquelles viennent reposer la paroi de l'oesophage et la paroi de la trachée.

4. Vanne de dérivation selon l'une quelconque des revendications 1 à 3, **caractérisée en ce que** l'enveloppe en matériau synthétique (4) est composée de silicone.
